Europäisches Patentamt

European Patent Office

Office européen des brevets

(11) Publication number: **0 108 508**
B1

# EUROPEAN PATENT SPECIFICATION

(45) Date of publication of patent specification: 07.02.90

(51) Int. Cl.⁵: **C 12 N 15/00,** C 12 P 21/00,
C 12 Q 1/04, G 01 N 33/00

(21) Application number: 83306098.1

(22) Date of filing: 07.10.83

(60) Divisional application 87103953 filed on 18.03.87.

(54) Hydrogen uptake gene cosmid and process of constructing same.

(30) Priority: 08.10.82 US 433503

(43) Date of publication of application:
16.05.84 Bulletin 84/20

(45) Publication of the grant of the patent:
07.02.90 Bulletin 90/06

(84) Designated Contracting States:
AT BE CH DE FR GB IT LI LU NL SE

(56) References cited:
NATURE, vol. 288, no. 5786, November 6, 1980,
New York, USA N.J. BREWIN et al. "Co-transfer
of determinants for hydrogenase activity and
nodulation ability in Rhizobium
leguminosarum" pages 77-79

Science, Vol. 203, 23 March 79, pp 1255-1257
J. Bacteriology Feb. 80, pp664-670, Vol. 141, No.
2

Proc. Nat. Acad. Sci. USA, vol. 76, no. 4, pp
1788-1799, April 79

J. Bacteriology, Jan 79, pp 153-160, vol. 137, no.
1

(73) Proprietor: **STATE OF OREGON, by and through
the Oregon State Board of Higher Education on
behalf of Oregon State University
Corvallis, Oregon 97330 (US)**

(72) Inventor: **Cantrell, Michael A.
421 S.W. 8th Corvallis
Oregon 97333 (US)**
Inventor: **Haugland, Richard A.
39-1 Westbrook Hills Drive
Syracuse NY 13215 (US)**
Inventor: **Evans, Harold J.
2939 N.W. Mulkey Corvallis
Oregon 97330 (US)**

(74) Representative: **Brown, John David et al
FORRESTER & BOEHMERT Widenmayerstrasse
4/I
D-8000 München 22 (DE)**

Courier Press, Leamington Spa, England.

# EP 0 108 508 B1

**Description**

## Summary of the invention

The present invention relates to nitrogen fixation by leguminous plants. More specifically, it relates to pure strains of nitrogen-fixing microorganisms that contain insert DNA to facilitate the oxidation of $H_2$ produced as a byproduct of nitrogen fixation and thereby increase the amount of energy available for the nitrogen fixation process.

To facilitate the fixation of nitrogen in leguminous plants, seeds are inoculated with desirable strains of nitrogen-fixing microorganisms. A variety of techniques are used, including the mixing of cultures with the soil where plants are to be grown. More often, seeds are coated with a medium that includes the desired nitrogen-fixing microorganism. When seeds with such a coating thereon germinate, the seedling plants are directly exposed to the microorganism.

Nitrogenases from legume nodules and all known sources catalyze an ATP-dependent reduction of not only $N_2$ to $NH_4$, but also protons to $H_2$. This $H_2$ production results in an inefficient use of the energy provided by the organism to the $N_2$-fixing process. Most $N_2$-fixing, free-living microorganisms, but a minority of strains of *Rhizobium*, possess the capacity for synthesis of an $H_2$-recycling system which oxidizes the $H_2$ produced during $N_2$ fixation, thus recapturing some of the energy expended during $H_2$ evolution. [Evans, H. J., Purohit, K., Cantrell, M. A., Eisbrenner, G., Russel, S. A., Hanus, F. J., and Lepo, J. E. 1981, *Current Perspectives in Nitrogen Fixation*, ed. Gibson, A. H. and Newton, W. E. (Elsevier/North Holland, New York, NY), 84—96].

Dixon [Dixon, R. O. D. 1978. *Biochimie* 60, 233—236] pointed out several potential advantages of $H_2$-oxidizing capability to $N_2$-fixing organisms. Subsequent investigations have shown that an active $H_2$-oxidizing system in *R. japonicum* can increase the ATP content of bacteroid cells and also provide a mechanism for additional protection of bacteroid nitrogenase from $O_2$ inactivation [Emerich, D. W., Ruiz-Argüeso, Ching, T. M., and Evans, H. J. 1979. *J. Bacteriol.* 137, 153—160].

In glasshouse and field experiments, soybean plants inoculated with groups of hydrogenase positive (Hup$^+$) strains of *R. japonicum* were reported to contain higher percentages of N in their tissues and seeds than plants inoculated with groups of hydrogenase negative (Hup$^-$) strains [Albrecht, S. L., Maier, R. J., Hanus, F. J., Russell, S. A., Emerich, D. W., and Evans, H. J. 1979, *Science* 203, 1255—1257], [Hanus, F. J., Albrecht, S. L., Zablotowicz, R. M., Emerich, D. W., Russell, S. A., and Evans, H. J. 1981, *Agron. J.* 73, 368—372]. The strains of *R. japonicum* with highly active $H_2$-oxidizing capacities have been shown to grow as chemolithotrophs using $H_2$ and $CO_2$ as their sources of energy and carbon, respectively [Hanus, F. J., Maier, R. J., & Evans, H. J. (1979) *Proc. Natl. Acad. Sci. USA* 76, 1783—1792].

$H_2$ is oxidized by the hydrogenase enzyme and $CO_2$ is fixed by a RuBP carboxylase [Lepo, J. E., Hanus, F. J., and Evans, H. J. (1980) *J. Bacteriol.* 141, 664—670]. Strains which possess such chemolithotrophic capacity may have an increased chance of survival in the soil where there is a limitation of fixed carbon substrates.

Highly active hydrogen-oxidizing systems have been found only in about 25% of *R. japonicum* strains and in no strain of *R. trifolii* or *R. meliloti* [Evans, H. J., Purohit, K., Cantrell, M. A., Eisbrenner, G., Russell, S. A., Hanus, F. J., & Lepo, J. E. (1981) *Current Perspectives in Nitrogen Fixation*, eds. Gibson, A. H. & Newton, W. E., (Elsevier/North Holland, New York, NY) 84—96]. Most strains of *R. leguminosarum* are Hup$^-$ and with a few exceptions the Hup$^+$ strains of this species recycle only a small proportion of the $H_2$ produced by the nitrogenase reaction [Nelson, L. M. and Salminen, S. O. (1982) *J. Bacteriol.* 151, 989—995]. It would be beneficial if Hup$^-$ strains of any one of the several species of *Rhizobium* could be modified to Hup$^+$, e.g. by a transfer of the genes for the Hup phenotype from highly active Hup$^+$ strains. Beneficial effects of the transfer of genes for the Hup phenotype could include not only the ability to utilize $H_2$ *per se* but also the ability to grow chemolithotrophically.

At least some of the determinants for the Hup phenotype have been shown to be plasmid-borne and transmissible in *Alcaligenes eutrophus* [Friedrich, B., Hogrefe, C., & Schlegel, H. G. (1981) *J. Bacteriol.* 147, 198—205] and in *R. leguminosarum* [Brewin, N. J., DeJong, T. M., Phillips, D. A. and Johnston, A. W. B. (1980) *Nature* 288, 77—79]. In both *R. leguminosarum* and *A. eutrophus*, the Hup phenotype was carried on large, naturally occurring plasmids which must carry many other genes. The plasmid carrying Hup determinants in *R. leguminosarum* also was shown to carry determinants for nodulation specificity and for $N_2$ fixation. The host range for this plasmid may be limited [Brewin, N. J., DeJong, T. M., Phillips, D. A. & Johnston, A. W. B. (1980) *Nature 288*, 77—79], and recipients of this plasmid may only be able to form effective nodules on pea plants.

Techniques for measurement of $H_2$ uptake have been described [Hanus, F. J., Carter, K. R. & Evans, H. J. (1980) *Methods Enzymol.* 69, 731—739] [Bethlenfalvay, G. J. & Phillips, D. A. (1979) *Plant Physiol.* 63, 816—820]. Colonies of *Azotobacter chroococcum* have been screened for expression of hydrogenase activity by observations of differences in rates of reduction of methylene blue by Hup$^+$ and Hup$^-$ *Azotobacter* colonies [Postgate, J. R., Partridge, C. D. P., Robson, R. L., Simpson, F. G., & Yates, M. G. (1982) *J. Gen. Microbiol.* 128, 905—908]. But none of these methods relates to the isolation of Hup$^+$ *Rhizobium* colonies from a large number of colonies.

A procedure for the introduction of genetic information for hydrogen-uptake systems into a broad host range, transmissible DNA replicon has now been developed. A definitive method of screening large

2

numbers of bacterial colonies for the necessary isolation of strains carrying determinants for hydrogenase expression also has now been discovered. Two products of these procedures, pHU1 and pHU2 DNA's, contain hydrogen-uptake genes from *Rhizobium japonicum* strain 122 DES and have been transferred from *Escherichia coli* to the Hup⁻ *R. japonicum* mutant strains PJ17nal and PJ18nal whereupon they converted free-living cells of these strains, most likely by genetic complementation in the first instance and by recombination in the second instance, to a Hup⁺ phenotype. Both pHU1 and pHU2 can give bacteroids of PJ17nal, occurring in soybean nodules, sufficient hydrogen-uptake activity to utilize all hydrogen gas produced in the nodules. These products and other products constructed by the described procedure may be used to introduce genetic information for a hydrogen-uptake system into strains of *Rhizobium* currently lacking such systems. Expression of the introduced genetic information in Hup⁻ recipient *Rhizobium* strains will increase the amount of energy available for nitrogen fixation.

According to one aspect of the present invention, there is provided a cosmid pHU1, being a cosmid produced by recombinant DNA techniques and containing HU DNA, which DNA contains *hup* genes or portions of *hup* genes, which cosmid pHU1 is present in the culture of *Escherichia coli* HB101 having a deposit accession number ATCC 39195.

According to a further aspect of the present invention there is provided a cosmid pHU2, being a cosmid produced by recombinant DNA techniques and containing HU DNA, which cosmid pHU2 is present in the culture of *Escherichia coli* HB101 having deposit accession number ATCC 39196.

Cosmid pHU1 comprises cosmid pLAFR1, being a broad host range cloning vehicle, joined with HU DNA from *Rhizobium japonicum* and which upon digestion with the restriction endonucleases listed in Table II separates into fragments of the molecular sizes identified in Table II.

Cosmid pHU2 comprises cosmid pLAFR1, joined with HU DNA from *Rhizobium japonicum* and which upon digestion with the restriction endonucleases *Eco*RI separates into fragments of the molecular sizes identified in Table II.

According to a further aspect of the present invention, there is provided a replicon which can be maintained in a *Rhizobium* species that is useful for nitrogen fixation in leguminous plants, the replicon is a cloning vector joined with HU DNA necessary for hydrogen-uptake activity, the HU DNA being of the type present in cosmid pHU1 or cosmid pHU2.

According to a further aspect of the present invention, there is provided a *Escherichia coli* HB101, containing the cosmid pHU1, having the deposit accession number ATCC 39195.

According to a further aspect of the present invention, there is provided a *Escherichia coli* HB101, containing the cosmid pHU2, having the deposit accession number ATCC 39196.

According to a further aspect of the present invention, there is provided a method for producing a replicon which contains genes or portions of genes coding for hydrogen gas uptake capacity (Hup) and which is suitable for introduction into a nitrogen-fixing *Rhizobium* species, the method comprising: joining fragments of DNA which contain *hup* genes or portions of *hup* genes, of the type present in a cosmid pHU1 or a cosmid pHU2, to replicons which can exist in a Hup⁻ strain of a *Rhizobium* species; introducing the replicons into cells of the Hup⁻ strain; identifying which samples of Hup⁻ strain have been converted to Hup⁺; isolating the replicon; and introducing the replicon into other species.

According to a further aspect of the present invention, there is provided a process for providing an active hydrogen-oxidizing system in free-living cells of a strain of a *Rhizobium* species that lack such a system and that is useful for nitrogen fixation in a leguminous plant comprising inserting a pHU1 cosmid or a pHU2 cosmid into the cells.

Preferably this process further comprises the step of identifying a colony of *Rhizobium japonicum* having hydrogen-uptake activity from a group of colonies, some of which do not have hydrogen-uptake activity, which step comprises: replicating colonies of the group onto a surface of a porous, sterile support material; incubating cells on the surface under conditions that derepress their hydrogenase; treating the support material with a methylene blue indicator solution containing inhibitors of endogenous respiration; maintaining the support material with attached colonies in an H₂ atmosphere whereupon colonies with hydrogen uptake activity reduce the methylene blue to its leuco form; extracting and/or cloning HU DNA from the colonies having hydrogen up-take activity and producing a cosmid pHU1 or a cosmid pHU2, prior to inserting the pHU1 or pHU2 cosmid into the cells.

More preferably the support material comprises filter paper. During the incubation, the filter paper is preferably placed colony side up on plates of Repaske's medium. The incubation may occur in a closed container wherein the atmosphere is initially about 5% H₂, 5% CO₂, 0.7% O₂, and the remainder N₂, the level of O₂ being allowed to decrease to below 0.1% during incubation; the level of O₂ thereafter is preferably maintained at about 0.2% by additions of O₂. The preferred methylene blue indicator solution contains 200 mM iodoacetic acid, 200 mM malonic acid, 10 mM methylene blue, 50 mM KH₂PO₄, 2.5 mM MgCl₂, and an amount of KOH sufficient to adjust the pH to 5.6.

According to a further aspect of the present invention, there is provided a process for enhancing nitrogen fixation in leguminous plants that harbor nitrogen-fixing bacteria normally lacking an active hydrogen-oxidizing system, the process comprising: identifying a strain of a *Rhizobium* species which has desired nitrogen-fixing activity when present in the roots of plants of a particular Leguminosae species; introducing DNA containing hydrogen-uptake gene material of the type present in a cosmid pHU1 or a cosmid pHU2 into free-living cells of the strain so that they are converted to Hup$^+$ phenotype; and inoculating such plants of the particular Leguminosae species with such cells having Hup$^+$ phenotype.

Preferably, the *Rhizobium* species is *Rhizobium japonicum*; and the Leguminosae species is *Glycine max*.

More preferably, the DNA is introduced into the free-living cells by means of a pHU1 cosmid or a pHU2 cosmid.

According to a further aspect of the present invention there is provided a recombinant DNA molecule comprising a first segment containing HU DNA present in cosmid pHU1 or cosmid pHU2 flanked by second and third DNA segments that are not the DNA that flanks the HU DNA in naturally occurring organisms.

The process of this invention employs recombinant DNA techniques in conjunction with a screening method to allow production and isolation of recombinant replicons containing DNA which codes for genes specific for the hydrogen-uptake phenotype. These replicons are referred to herein as pHU DNA's. Specific examples of such replicons are the cosmids pHU1 and pHU2 described below. Section I below is a detailed description of a process for production and isolation of pHU DNA. Section II is a detailed description of recombinant cosmids made by the process.

The following definitions may be useful in understanding the terminology of this disclosure:

Replicon.—A genetic element which is capable of independent replication. This may include a plasmid, cosmid, or bacteriophage DNA.

Hup$^+$.—A designation for the phenotype of any organism having the ability to show uptake of hydrogen gas—synonymous with hydrogen gas oxidation and hydrogen uptake.

Hup$^-$.—A designation for the phenotype of any organism which does not have the ability to show hydrogen uptake.

*hup.*—A designation for a hydrogen uptake gene.

HU DNA.—DNA which contains *hup* genes or portions of *hup* genes.

pHU DNA.—A replicon produced by recombinant DNA techniques, which contains HU DNA.

I. Production and isolation of pHU DNA

An object of the present process is to isolate and produce DNA which can be used to provide hydrogen uptake activity to Hup$^-$ *Rhizobium* strains and in particular DNA which returns hydrogen uptake activity to certain specific Hup$^-$ test strains of *R. japonicum*. Such DNA may come from any organism which contains *hup* genes capable of either showing complementation to the Hup$^-$ test strains of *R. japonicum* or showing recombination with these strains of *R. japonicum*, thus converting them to a Hup$^+$ phenotype. The vector DNA to which the *Hup* gene-containing DNA (the insert DNA) is attached may be any replicon which can be introduced and maintained in *R. japonicum*.

A process according to this invention includes a number of steps, including one or more of the following:

A. Growth of bacterial strains and isolation of DNA.
B. Construction of insert DNA/vector DNA recombinants.
C. Introduction of recombinant DNA into recipient strains.
D. Identification of recipients containing pHU DNA.
E. Isolation of pHU DNA.

Step D is of particular interest in that it involves a procedure to distinguish colonies of the Hup$^+$ phenotype from thousands of colonies of *R. japonicum*. Colonies are grown on plates of any standard culture medium and then replicated onto a porous, sterile support material (for example, filter paper discs) and incubated under conditions which will derepress the hydrogen-uptake system. After an appropriate period of time, the discs are soaked in a solution containing methylene blue and inhibitors of endogenous respiration. The concentration of inhibitors should be sufficient to block methylene blue dye reduction via endogenous respiration. When the discs are then incubated under a stream of $H_2$, only colonies with an active hydrogenase (Hup$^+$) will show $H_2$-dependent methylene blue dye reduction as evidenced by conversion of the methylene blue in that area of the disc to a colorless form.

A. Growth of bacterial strains and isolation of DNA

Strains of *E. coli*, unless otherwise indicated are grown on LB medium at 32°C [Kahn, M., et al (1979) *Methods Enzymol.* 68, 268—280, incorporated herein by reference]. *R. japonicum* are routinely cultured in broth or an agar plates using the yeast extract mannitol medium described by Vincent [Vincent, J. M. (1970) *A Manual for the Practical Study of Root-Nodule Bacteria* (Blackwell, Oxford, UK) 59—60, incorporated herein by reference] or the hydrogen uptake medium described by Maier, et al [Maier, R. J., et al. (1978) *Prose; Natl. Acad. Sci., USA* 75, 3258—3262], adjusted to pH 7.0 and modified by adding 0.2 g KH$_2$PO$_4 \cdot$ H$_2$O and 0.03 g. NaH$_2$PO$_4 \cdot$ H$_2$O per liter instead of 0.15 g NaH$_2$PO$_4 \cdot$ H$_2$O. Strains of *E. coli* and *R. japonicum* used are listed in Table I. The cosmid, pLAFR1, was produced by Friedman et al [Friedman, A. M. Long, S. R., Brown, S. E., Buikema, W. J., & Ausubel, F. M. (1982) *Gene* 18, 289—296, incorporated herein by reference] from the plasmid, pRK290, which was produced by G. Ditta, S. Stranfield, D. Corbin, and D. R. Helinski [(1980), *Proc. Natl. Acad. Sci., USA* 77, 7347—7351, incorporated herein by reference] and is a broad host range cloning vehicle. The nal$^r$ derivatives of SR, PJ17 and PJ18 listed in Table I are spontaneous nalidixic acid-resistant mutants obtained by standard techniques.

TABLE I
Bacterial strains

| Strain | Relevant characteristics | Source or reference |
|---|---|---|
| *E. coli* | | |
| HB101 | *recA⁻, hsdR, hsdM, pro, leu*, Str$^r$ | 1 |
| HB101 (pLAFR1) | contains pLAFR1 | 2 |
| HB101 (pRK2013) | contains pRK2013 | 3 |
| BHB 2688 | N205, *recA, (λimm434, clts, b2, red3, Eam4, Sam7)/λ* | 4 |
| BHB2690 | N205, *recA⁻, (λimm434, clts, b2, red3, Dam15, Sam7)/λ* | 4 |
| *R. japonicum* | | |
| 122 DES | Hup$^+$, derivative of USDA 122 | 5 |
| SR | Hup$^+$, Str$^r$, Kan$^r$, derivative of 122 DES | 6 |
| SRnal | Nal$^r$ derivative of SR | 7 |
| PJ17 | Hup$^-$, Str$^r$, Kan$^r$, derivative of SR | 8 |
| PJ17nal | Nal$^r$ derivative of PJ17 | 9 |
| PJ18 | Hup$^-$, Str$^r$, Kan$^r$, derivative of SR | 8 |
| PJ18nal | Nal$^r$ derivative of PJ18 | 10 |

1. Boyer, H. B., et al. (1969) *J. Mol. Biol.* 41, 459—472; obtainable from ATCC 39195 and 39196.
2. Friedman, A. M., et al. (1982) *Gene* 18, 289—296.
3. Ditta, G., et al. (1980) *Proc. Natl. Acad. Sci. USA* 77, 7347—7351.
4. Hohn, B. (1979) *Methods Enzymol.* 68, 299—309.
5. Ruiz-Argüeso, T., et al. (1979) *Arch. Microbiol.* 121, 199—206.
6. Maier, R. J., et al. (1978) *Nature* 276, 494—495.
7. Cantrell, M. A., et al. (1983) *Proc. Natl. Acad. Sci. USA* 80, 181—185.
8. Lepo, J. E., et al. (1981) *J. Bacteriol.* 146, 614—620.
9. ATCC 39194.
10. ATCC 39193.

Each of the writings mentioned in the above notes is incorporated herein by reference.

Large scale preparations of cosmid DNA from *E. coli* and *R. japonicum* are obtained by the procedure of Cantrell, Hickok and Evans [(1982) *Arch. Microbiol* 131, 102—106, incorporated herein by reference]. For use in preparation of the clone bank, pLAFR1 DNA from *E. coli* is further purified by two CsCl/ethidium bromide gradient centrifugations in 49% (w/w) CsCl and 0.35 mg/ml ethidium bromide in a buffer solution at pH 8.0 containing 50 mM Tris-HCl, 20 mM EDTA (TE buffer). The initial refractive index of the gradient solution is 1.3910. Samples are centrifuged in a Beckman VTi65 rotor to equilibrium (12—16 hr) at 55,000 rpm. Covalently-closed circular DNA recovered from the second gradient centrifugation is diluted with TE buffer and pelleted by centrifugation in a Beckman SW60 rotor. The pelleted DNA is resuspended in 0.37 ml of a buffer containing 6 mM Tris-HCl, pH 7.4, 10 mM NaCl, 0.1 mM EDTA (DNA stroage buffer), extracted repeatedly with isoamyl alcohol and precipitated with ethanol. After drying the precipitate by evacuation at room temperature, it is redissolved and stored in 0.25 ml DNA storage buffer.

Small scale isolations of cosmid DNA from *E. coli* for use in restriction analyses are performed by use of a cleared lysate procedure [Kahn, M., Kolter, R., Thomas, C., Figurski, D., Meyer, R., Remaut, E., & Helinski, D. R. (1979) *Methods Enzymol.* 68, 271—272, incorporated herein by reference]. Total genomic DNA from 500 ml cultures of *R. japonicum* grown in HUM broth medium to an optical density at 540 nm of approximately 1.0 is isolated by a previously described procedure [Haugland, R. A., & Verma, D. P. S. (1981) *J. Mol. Appl. Genet. 1*, 205—217, incorporated herein by reference] with the following modification. After the spooled, ethanol-precipitated DNA is dried, it is redissolved in 5 ml of a pH 7.0 buffer solution containing 150 mM NaCl and 15 mM sodium citrate (1×SSC). A 1 mg/ml solution of RNase A in the same buffer is preheated 15 minutes at 80°C and then added to the DNA solution to a final concentration of 50 µg/ml. After a 30 minute incubation at 37°C, the DNA is again spooled onto a glass rod from an overlay of two volumes of cold ethanol and subsequently treated as referred to above.

B. Construction of insert DNA/vector DNA recombinants

*R. japonicum* DNA which is to be used as the insert DNA (HU DNA) is digested with the restriction enzyme *Eco*RI and size fractionated by the following procedure. *R. japonicum* 122 DES total DNA (200 µg) is incubated at 37°C with 20 µl of *Eco*RI (Bethesda Research Laboratories, Gaithersburg, MD, U.S.A.; 10 units/µl) for periods ranging from 20 to 115 minutes in 100 mM Tris-HCl, pH 7.2, 50 mM NaCl, 5 mM $MgCl_2$ and 2 mM β - mercaptoethanol. Aliquots from each *Eco*RI digestion time point are subjected to agarose gel electrophoresis by the method of Meyers *et al* [Meyers, J. A., Sanchez, D., Elwell, L. P., & Falkow, S. (1976) *J. Bacteriol.* 127, 1529—1537, incorporated herein by reference] with modifications of Haugland & Verma [Haugland, R. A., & Verma, D. P. S. (1981) *J. Mol. Appl. Genet*, 1, 205—207, incorporated herein by reference] to identify samples with average sizes of approximately 30 kb, 22 kb and 15 kb. Approximately 50 µg of DNA falling into each of these three classes are then pooled and extracted first with equal volumes of phenol (equilibrated with 100 mM Tris, pH 8.5) and then twice with double volumes of ether equilibrated as above. The DNA is then precipitated with 2 volumes of ethanol, incubated more than 30 minutes at −20°C, pelleted by centrifugation for 15 minutes in an Eppendorf microfuge, washed with cold 80% ethanol, recentrifuged as above, dried under vacuum and finally redissolved in 0.7 ml of a buffer containing 20 mM Tris-HCl, 10 mM EDTA, 50 mM NaCl, pH 8.0 (sucrose gradient buffer). The sample is heated for 10 minutes at 65°C and then layered on a sucrose gradient and centrifuged using the conditions described by Ditta *et al* [Ditta, G., Stanfield, S., Corbin, D., and Helinski, D. R. (1980) *Proc. Natl. Acad. Sci. USA* 77, 7347—7351, incorporated herein by reference]. Aliquots from 0.7 ml fractions taken from the bottom of the gradient are then subjected to agarose gel electrophoresis as described above to identify the fractions containing DNA in the size range of 12 to 30 kb. Fractions containing DNA in this size range are pooled and dialyzed at 4°C against several changes of 1/10-strength sucrose gradient buffer over a period of 24 hours. The dialyzed sample is then precipitated with two volumes of ethanol, pelleted by centrifugation at 15,000 RPM for 20 minutes in a Sorvall SS-34 rotor, washed with cold 80% ethanol, and recentrifuged as above, dried under vacuum and finally redissolved in 0.4 ml of DNA storage buffer.

The pLAFR1 DNA is digested with *Eco*RI under the conditions described above with the exception that *Eco*RI is added at a ratio of 5 units per µg of DNA, the mixture is incubated 90 minutes at 37°C, an additional 5 units of *Eco*RI per µg of DNA is added, the mixture is incubated an additional 90 minutes at 37°C, and the reaction is terminated by adding a volume of 0.25 M $Na_2$EDTA (pH 8.0) equal to 6% of the reaction volume to give a final concentration of 8 mM EDTA. The reaction mixture is extracted 2 times with equal volumes of redistilled phenol, then 3 times with equal volumes of ether, and traces of remaining ether are then removed by evaporation with a stream of $N_2$.

Ligation is performed as follows: 20 µg of pooled, partially digested 12—30 kb functions of *R. japonicum* DNA are added per µg of *Eco*RI-digested pLAFR1, the mixture is ethanol precipitated as described above, and the precipitate is redissolved in ligation buffer (66 mM Tris-HCl, pH 7.6, 6.6 mM $MgCl_2$). The resultant mixture of *Eco*RI-digested pLAFR1 and partially digested *R. japonicum* DNA is then ligated at concentrations of 0.05 and 1.0 mg/ml, respectively, in the presence of 700 U/ml T4 ligase (Bethesda Research Laboratories), 66 mM Tris-HCl, pH 7.6, 6.6 mM $MgCl_2$, 10 mM dithiothreitol and 66 µM ATP for 18 hours at 12°C.

C. Introduction of recombinant DNA into recipient strains

The recombinant DNA ligation mixture is packaged with an extract containing phage lambda head and tail components and the resultant packaged cosmids are adsorbed onto *E. coli* strain HB101.

Packaging extracts are prepared and DNA is packaged with the following modifications of the procedure of Blattner *et al.* [Blattner, F. R. Blechl, A. E., Denniston-Thompson, K., Faber, H. E., Richards, J. E., Slightom, J. L., Tucher, P. W., & Smithies, O. (1978) *Science* 202, 1279—1284, incorporated herein by reference] using the lambda lysogenic strains of *E. coli* BHB2688 and BHB2690 (see Table I). These lambda lysogens are each grown as 500 ml cultures in LB medium in a 2 l flask at 32°C to approximately $3 \times 10^8$ cells per ml. The cultures are heated in a water bath to 45°C, then maintained at that temperature for 30 minutes with constant agitation. They are then placed on a shaker at 37°C for 1 hour and then chilled on ice. All succeeding production of the extracts is at 4°C.

Production of the BHB2688 extract

Cells from three 500 ml cultures of BHB2688 are each centrifuged 10 minutes at 9000 rpm in a Sorvall GSA rotor and the pellets are resuspended in a total of 3 ml of a buffer containing 10% sucrose, 50 mM Tris-HCl, pH 7.4. A total of 150 µg of highly purified lysozyme (Sigma) is then added in a volume of 75 µl of 0.25 M Tris-HCl, pH 7.4. The sample is mixed gently and frozen in liquid $N_2$. It is then thawed on ice, 400 µl of the M1 buffer of Blattner *et al.* is added, it is incubated 30—45 minutes at 4°C, and centrifuged 35 minutes at 28,000 rpm in a Beckman type 30 rotor. The BHB2688 extract consists of aliquots of the supernatant stored at −70°C.

Production of the BHB2690 extract

Cells from one culture of 500 ml of BHB2690 are centrifuged 10 minutes at 9000 rpm in a Sorvall GSA rotor and the pellet is resuspended in 3.1 ml of the buffer A of Blattner *et al*. The suspension is sonicated as described by Blattner, *et al*. and centrifuged 5 minutes at 7000 rpm in a Sorvall SS-34 rotor. The BHB2690 extract consists of aliquots of the supernatant stored at −70°C.

Packaging of DNA and transfer into *E. coli*

DNA packaging is initiated by addition of the following in the order indicated: 7 µl of the buffer A of Blattner *et al*, 3 µl of ligated DNA, 1 µl of the M1 buffer of Blattner *et al*, 5 µl of BHB2690 extract and 5 µl of BHB2688 extract. After incubation for 1 hour at 23°C, a drop of $CHCl_3$ is added, the mixture is serially diluted, and transduction with strain HB101 is performed as described by Hohn [Hohn, B. (1979) *Methods Enzymol.* 68, 299—309, incorporated herein by reference].

Selection for tetracycline-resistant transductance on LB plates with 20 µg/ml of tetracycline results in the growth of a large number of colonies which constitute a gene bank. During generation of the gene bank which lead to isolation of pHU1 and pHU2, tetracycline resistant transductants were obtained at a frequency of $7.8 \times 10^5$ per µg of vector DNA. The resultant gene bank contained more than 40,000 clones. Analysis of 24 of these clones chosen at random has shown that 83% contain insert DNA with an average molecular size of 22.6 kb.

PJ17nal is an *R. japonicum* revertible Hup⁻ mutant, ATCC 39194. This biologically pure culture is available from the permanent collection of the American Type Culture Collection, Rockville, Maryland, U.S.A. Colonies from the clone bank were conjugated *en masse* with PJ17nal by use of the triparental mating system of Ditta *et al* [Ditta, G., Stanfield, S., Corbin, D., & Helinski, D. R. (1980) *Proc. Natl. Acad. Sci. USA* 77, 7347—7351, incorporated herein by reference]. In this procedure, cultures of *E. coli* containing recombinant cosmids and *E. coli* containing the mobilization helper plasmid pRK2013, both grown to early log phase (approximately 0.4 absorbance units at 600 nm) in LB medium are each concentrated by centrifugation at 8000 rpm in a Sorvall SS-34 rotor and resuspended in 0.05 vol of YEM medium. A culture of *R. japonicum* recipient cells, grown to late log phase (approximately 0.5 absorbance units at 540 nm) in YEM medium is likewise concentrated. Aliquots of 0.1 ml of each of these three cell suspensions (approximately $5 \times 10^8$ *R. japonicum* cells and approximately $5 \times 10^7$ of each of the two *E. coli* cell types) are then spread together on a YEM medium agar plate at pH 7.0, excess liquid is removed by evaporation under a sterile, laminar-flow hood, and the cells are then incubated at 29°C for 3 to 4 days. After this time, the cells are scraped off the plate and suspended in 8 ml of the mineral salts solution used for the HUM medium containing 0.01% Tween 80. Dilutions ranging from $10^{-1}$ to $10^{-6}$ are plated on either YEM or HUM medium with 100 µg/ml tetracycline and 100 µg/ml nalidixic acid. Recipient colonies containing pHU DNA are then identified from plates containing up to 3000 transconjugant colonies.

D. Identification of recipients containing pHU DNA

Recipient colonies which have received DNA from the clone bank (transconjugants) are replicated onto sterile filter paper discs (Whatman 541) which are then placed colony side up on plates of Repaske's medium [Repaske, R., & Repaske, C. (1976) *Appl. Env. Microbiol*, 32, 585—591; Repaske, R., & Mayer, R. (1976) *Appl. Env. Microbiol.* 32, 592—597, both incorporated herein by reference].

Cells on the filters are then derepressed by incubation for 4 to 5 days in an atmosphere which initially contains by volume 5% $H_2$, 5% $CO_2$, approximately 0.7% $O_2$, and the remainder $N_2$. $O_2$ is consumed such that levels of $O_2$ gradually decrease to less than 0.1% of the gas volume during incubation and are subsequently maintained at about 0.2% by additions of $O_2$. In cases where many Hup⁺ colonies are present during the derepression period, $H_2$ levels also decrease as determined by gas chromatography and are subsequently maintained at approximately 5% by additions of $H_2$.

Filters with depressed colonies are removed from the Repaske plates and soaked in 0.8 ml of a solution containing 200 mM iodoacetic acid (Sigman Chemical Co., St. Louis, MO, U.S.A.), 200 mM malonic acid, 10 mM methylene blue (Nutritional Biochemical Co.), 50 mM $KH_2PO_4$, 2.5 mM $MgCl_2$ adjusted to pH 5.6 with KOH. After approximately 15 min, the plates are tilted slightly and excess solution is removed from the sides of the filters with a Pasteur pipette. An additional 45 min in air is allowed for the dye/inhibitor solution to equilibrate with the colonies, and the plates with filters and attached colonies are transferred to a Plexiglass tray (inside dimensions: 29×39×3 cm) with an outer well filled with water. A Plexiglass cover with side walls that fit into the outer well of the tray is used to form a partial seal against entry of air into the tray. The cover also contains two portals to permit controlled entry and exit of cylinder gases.

$N_2$ is flushed through the chamber for 15 minutes to reduce the $O_2$ concentration over the filters. The chamber is placed in a fume hood for safety purposes and $H_2$ (passed through a catalytic $O_2$ purifier—Engelhard Industries, Inc.) is then introduced at a flow rate of approximately 150 ml per minute. Both gases are passed through $H_2O$ before entering the Plexiglass chamber in order to reduce evaporation from the filters. Colonies with $H_2$ uptake activity reduce methylene blue to its leuco form within 0.5 to 3 hours while Hup⁻ colonies do not reduce methylene blue during periods of 20 hours and more. Hup⁺ colonies are found amongst the population of Hup⁻ recipients at frequencies of approximately $10^{-2}$ to $10^{-3}$. The Hup⁺ colonies are then isolated from the master plates and pHU cosmid DNA is isolated as described below.

E. Isolation of pHU DNA

Colonies showing methylene blue reduction activity in the initial screening are picked from the original plates from which the filter replicas were taken and streaked for single colonies on HUM medium agar plates containing 100 µg/ml each of tetracycline and nalidixic acid. When colonies on these plates have grown to maximum size (10—15 days), the plates are replicated onto filter paper discs and the portions of the colonies transferred are derepressed and subjected to the above-described methylene blue reduction screening procedure for the identification of recipients containing pHU DNA. Isolated colonies identified as having dye reducing activity in this second screening are picked from the master plates and used to inoculate 200 ml YEM broth cultures containing 45 µg/ml tetracycline. The cultures are grown to an optical density of approximately 0.2 absorbance units at 540 nm and the cells then harvested and subjected to the plasmid isolation procedure of Cantrell *et al.* [(1982) *Arch. Microbiol.* 131, 102—106]. Partially purified cosmid DNA obtained by this procedure is then used to transform *E. coli* HB101 cells by the procedure of Davis *et al* [Davis, R. W., Botstein, D., & Roth, J. R. (1980) *A Manual for Genetic Engineering: Advanced Bacterial Genetics* (Cold Spring Harbor Laboratory, Cold Spring Harbor, NY) 140—141, incorporated herein by reference] with the modification that cryogenically stored, competent *E. coli* cells, prepared by the method of Morrison [Morrison, D. A. (1979) *Methods Enzymol.* 68, 326—331, incorporated herein by reference] are used as recipients. The transformed *E. coli* are selected on LB medium with 20 µg/ml tetracycline.

The resultant transformants are used to purify pHU cosmid DNA by standard techniques. For example, the above-described procedures for growth of bacterial strains and isolation of DNA are used and the procedure of Guerry, LeBlanc, and Falkow [*J. Bacteriol.* (1973) 116, 1064—1066] followed by CsCl/EtBr gradient centrifugation is used.

II. Characteristics of pHU1 and pHU2

Two cosmids isolated by the above procedures are pHU1 and pHU2. pHU1 is obtainable from *Escherichia coli* HB101 (pHU1), ATCC 39195. pHU2 is obtainable from *Escherichia coli* HB101 (pHU2), ATCC 39196. These biologically pure cultures are available from the permanent collection of the American Type Culture Collection, Rockville, MD, U.S.A. The replicons are unique because they contain HU DNA which is attached by recombinant DNA techniques to vector DNA. The specific vector DNA used (pLAFR1) is not itself new. Other vector DNA may be similarly usable so long as it can be easily introduced and maintained in *R. japonicum* and a bacterium such as *E. coli*, commonly used in genetic laboratories. The HU DNA is the insert DNA which produces the Hup⁺ character in the recipient test strains as described below. The HU DNA may come from any organism which contains *hup* genes. The characteristics listed below should not be construed as limiting, nor need the functional characteristics be specifically associated with DNA fragments having the molecular sizes listed.

A. Physical characteristics of pHU1 and pHU2

Both the cosmids pHU1 and pHU2 are approximately 47 kb in molecular size and thus have a molecular weight of approximately $3 \times 10^7$. The approximate molecular sizes in kilobase pairs of DNA fragments produced from restriction endonuclease digestion of pHU1 and pHU2 with *Eco*RI and a combination of *Eco*RI and *Bgl*II are given in Table II. Fragments designated by asterisks in the table occur in the cosmid vector pLAFR1 and for this reason are not fragments of HU DNA. Results given in the table were obtained by digesting pHU1 and pHU2 for two hours at 37° with 5.5 units *Eco*RI (Bethesda Research Laboratories, Inc.) per µg DNA and/or 5 units *Bgl*II (Boerhinger Mannheim, Inc.) per µg DNA. The buffer used for all digestions contains 50 mM Tris, pH 8.0, 10 mM MgCl₂, 50 mM NaCl and 2 mM β - mercaptoethanol. Following restriction endonuclease digestions, the DNA's were electrophoresed in a 0.8% agarose gel made up in a buffer containing 89 mM Tris, 2.5 mM EDTA and 89 mM boric acid, pH 8.3. Additional fragments produced in the restriction digests with sizes less than 0.4 kilobase pairs would not have been detected in these analyses.

The DNA used as a source of HU DNA (*R. japonicum* 122 DES DNA) was originally digested with the restriction enzyme *Eco*RI. As a result, the HU DNA present in pHU1 and pHU2 could be easily cleaved from the vector, pLAFR1, by partial digestions with *Eco*RI by procedures well known in the art. Commonly used procedures would then allow joining of the HU DNA with different replicons so that pHU insert DNA can be transferred to many species of bacteria. Because pLAFR1 is a wide host range vector, pHU1 and pHU2 can be inserted in multiple species of bacteria whereby, for example, pHU insert DNA can be easily maintained in *E. coli*, transferred from *E. coli* to *R. japonicum*, and maintained in the *R. japonicum*.

TABLE II

Restriction fragment sizes in Kb pairs of pHU1 and pHU2 DNA's

| | pHU1 | | pHU2 |
| --- | --- | --- | --- |
| | Digestion with *Eco*RI | Digestion with *Eco*RI+*Bgl*II | Digestion with *Eco*RI |
| 21.6* | 19.0* | | 21.6* |
| 13.0 | 7.6 | | 13.0 |
| 5.0 | 5.0 | | 4.8 |
| 2.9 | 2.9 | | 2.9 |
| 2.3 | 2.7 | | 2.3 |
| 1.55 | 1.64* | | 2.2 |
| 0.60 | 1.55 (doublet) | | 0.60 |
| | 1.51 | | |
| | 1.09* | | |
| | 0.95 | | |
| | 0.68 | | |
| | 0.60 | | |

B. Functional characteristics of pHU1 and pHU2

Conjugation of HB101 containing pHU1 or pHU2 with *R. japonicum* PJ17nal by the procedures described above results in introduction of the cosmids into PJ17nal. Derepressed cells of *R. japonicum* strain PJ17nal (see Section I, Part D above for derepression procedure) containing pHU1 or pHU2 DNA's exhibit both methylene blue and $O_2$-dependent $H_2$ uptake activity. The aforementioned activities occur as a result of genetic complementation of a mutated DNA sequence present within strain PJ17nal by insert DNA sequences occurring with the HU1 or HU2 DNA's and are comparable with the activities demonstrated by derepressed free-living cells of the Hup$^+$ *R. japonicum* strain 122 DES. Derepressed cells of PJ17nal containing the vector pLAFR1 show no $H_2$-uptake activity.

$O_2$-dependent $H_2$-uptake activity is measured by the following method. *R. japonicum* cells are grown to an absorbance (540 nm) of 0.25 in 8 ml of YEM medium (containing 50 µg/ml of tetracycline when strains with plasmids are used). Cells are harvested by centrifugation and cell pellets resuspended in 0.2 ml of HUM medium. The suspensions are spread on plates of Repaske's medium and derepressed for 4 days under the gas mixture described for the methylene blue colony screen. Cells are removed from the plates and suspended in 5 ml of 50 mM $KH_2PO_4$ and 2.5 mM $MgCl_2$ buffer adjusted to pH 7.0 with KOH. The suspensions are diluted 10-fold in the above buffer and assayed amperometrically for rates of $O_2$-dependent $H_2$ uptake by the method of Hanus, Carter and Evans [(1980) *Methods Enzymol.* 69, 731—739, incorporated herein by reference].

Using the growth conditions and procedures described below, soybeans infected with PJ17nal containing pHU1 and pHU2 DNA's can form nodules which demonstrate no $H_2$ evolution. In a nodulation test which was done as described below, soybean plants infected with PJ17nal containing pHU1 or pHU2 formed nodules which demonstrated no $H_2$ evolution. Acetylene reduction activities by these nodules and those formed by strain 122 DES after similar inoculations and growth of soybeans are comparable. The pHU insert DNA does not interfere with the ability of PJ17nal to effectively nodulate soybean plants and likely will not interfere with the ability of any recipient *Rhizobium* species to effectively nodulate its natural plant host.

For plant tests, soybean seed [*Glycine max* (L.) Merr., cultivar Wilkin] are surface disinfected by the following procedure. Seeds are soaked in 70% ethanol for 10—20 seconds, rinsed 5—8 times with sterile distilled water, incubated 3 minutes in a 2% solution of sodium hypochlorite, then rinsed 5—8 times more with sterile distilled water and germinated on plates of 0.8% agar, containing one-half strength N-free nutrient solution [Harper, J. E. & Nicholas, J. C. (1976) *Physiol. Plant* 38, 24—28, incorporated herein by reference] to which is added 3 mM $CaSO_4$. After 48 hours, seedlings are immersed for 20 minutes in 5

9

day-old YEM broth cultures (with 45 µg/ml tetracycline for transconjugants) of desired strains of *R. japonicum*. The inoculated seedlings are transferred to a sterile mixture of equal parts sand and vermiculite in 250 ml flasks. Seedlings are protected by cotton plugs and placed in a growth cabinet (16 hr day—8 hr night cycle) at an irradiance of about 25 µE · m$^{-1}$ · sec$^{-1}$. The day and night temperatures are maintained near 28°C and 23°C, respectively. Plants receive sufficient sterile one-half strength N-free nutrient solution [Harper, J. E., & Nicholas, J. C. (1976) *Physiol. Plant* 38, 24—28, incorporated herein by reference] to maintain a moist medium without allowing free solution in the flasks. Plants are harvested 30 days after germination and nodules are assayed for rates of $C_2H_2$ reduction and $H_2$ evolution and for bacteroid hydrogenase activity by procedures used by Lepo *et al* [Lepo, J. E., Hickok, R. E., Cantrell, M. A., Russell, S. A., & Evans, H. J. (1981) *J. Bacteriol.* 146, 614—620, incorporated herein by reference].

PJ18nal is another *R. japonicum* revertible Hup$^-$ mutant, ATCC 39193. This biologically pure culture is available from the permanent collection of the American Type Culture Collection, Rockville, MD, U.S.A. Conjugation of HB101 (pHU1) or HB101 (pHU2) with PJ18nal allows introduction of the cosmids into PJ18nal. Methylene blue-dependent hydrogen-uptake activity is exhibited by colonies containing either pHU1 or pHU2 at frequencies of approximately 1—3×10$^3$.

These frequencies of appearance of Hup$^+$ colonies are calculated by dividing the number of Hup$^+$ tetracycline resistant colonies by the total number of tetracycline resistant colonies.

The strains PJ17nal and pJ18nal lack the ability to grow chemolithotrophically only because they are Hup$^-$ due to a single point mutation. The strain from which they were derived, SR, had the ability to grow chemolithotrophically. A number of PJ17nal transconjugants which were coverted to Hup$^+$ were identified by their ability to grow chemolithotrophically after introduction of pHU cosmids. The cosmids pHU1 and pHU2 were not analyzed in this manner, but since they convert PJ17nal to a Hup$^+$ character, they should also convert PJ17nal into a chemolithotroph.

While we have described and given examples of preferred embodiments of our inventions, it will be apparent to those skilled in the art that changes and modifications may be made without departing from our inventions in their broader aspects. We therefore intend the appended claims to cover all such changes and modifications as fall within the true spirit and scope of our inventions.

The features disclosed in the foregoing description, in the following claims and/or in the accompanying drawings may, both separately and in any combination thereof, be material for realising the invention in diverse forms thereof.

## Claims

1. A cosmid pHU1, being a cosmid produced by recombinant DNA techniques and containing HU DNA, which DNA contains *hup* genes or portions of *hup* genes, which cosmid pHU1 is present in the culture of *Escherichia coli* HB101 having a deposit accession number ATCC 39195.

2. A cosmid pHU2, being a cosmid produced by recombinant DNA techniques and containing HU DNA as defined in Claim 1, which cosmid pHU2 is present in the culture of *Escherichia coli* HB101 having deposit accession number ATCC 39196.

3. A replicon which can be maintained in a *Rhizobium* species that is useful for nitrogen fixation in leguminous plants, wherein the replicon is a cloning vector joined with HU DNA as defined in Claim 1 necessary for hydrogen-uptake activity, the HU DNA being the HU DNA present in cosmid pHU1 as defined in Claim 1 or cosmid pHU2 as defined in Claim 2.

4. *Escherichia coli* HB101, containing the cosmid pHU1 as defined in Claim 1, having the deposit accession number ATCC 39195.

5. A biologically pure culture of the *Escherichia coli* HB101 according to Claim 4.

6. *Escherichia coli* HB101, containing the cosmid pHU2 as defined in Claim 2, having the deposit accession number ATCC 39196.

7. A biologically pure culture of the *Escherichia coli* HB101 according to Claim 6.

8. A method for producing a replicon which contains genes or portions of genes coding for hydrogen gas uptake capacity (Hup) and which is suitable for introduction into a nitrogen-fixing *Rhizobium* species, the method comprising: joining fragments of DNA which contain *hup* genes or portions of *hup* genes, of the type present in a cosmid pHU1 as defined in Claim 1 or a cosmid pHU2 as defined in Claim 2, to replicons which can exist in a Hup$^-$ strain of a *Rhizobium* species; introducing the replicons into cells of the Hup$^-$ strain; identifying which samples of Hup$^-$ strain have been converted to Hup$^+$; and isolating the replicon.

9. A process for providing an active hydrogen-oxidizing system in free-living cells of a strain of a *Rhizobium* species that lack such a system and that is useful for nitrogen fixation in a leguminous plant comprising inserting a pHU1 cosmid as defined in Claim 1 or a pHU2 cosmid as defined in Claim 2 into the cells.

10. A process according to Claim 9, which process further comprises the step of identifying a colony of *Rhizobium japonicum* having hydrogen-uptake activity from a group of colonies, some of which do not have hydrogen-uptake activity, which step comprises: replicating colonies of the group onto a surface of a porous, sterile support material; incubating cells on the surface under conditions that derepress their hydrogenase; treating the support material with a methylene blue indicator solution containing inhibitors of endogenous respiration; maintaining the support material with attached colonies in an $H_2$ atmosphere whereupon colonies with hydrogen uptake activity reduce the methylene blue to its leuco form; extracting and/or cloning HU DNA as defined in Claim 1 from the colonies having hydrogen uptake activity and producing a cosmid pHU1 as defined in Claim 1 or a cosmid pHU2 as defined in Claim 2, prior to inserting the pHU1 or pHU2 cosmid into the cells.

11. The process of Claim 10, wherein: the support material comprises filter paper; during the incubating, the filter paper is placed colony side up on plates of Repaske's medium; the incubating occurs in a closed container wherein the atmosphere is initially about 5% $H_2$, 5% $CO_2$, 0.7% $O_2$, and the remainder $N_2$, the level of $O_2$ being allowed to decrease to below 0.1% during incubating; the level of $O_2$ thereafter is maintained at about 0.2% by additions of $O_2$; the methylene blue indicator solution contains 200 mM iodoacetic acid, 200 mM malonic acid, 10 mM methylene blue, 50 mM $KH_2PO_4$, 2.5 mM $MgCl_2$, and an amount of KOH sufficient to adjust the pH to 5.6.

12. A process for enhancing nitrogen fixation in leguminous plants that harbor nitrogen-fixing bacteria normally lacking an active hydrogen-oxidizing system, the process comprising: identifying a strain of a *Rhizobium* species which has desired nitrogen-fixing activity when present in the roots of plants of a particular Leguminosae species; introducing DNA containing hydrogen-uptake gene material of the type present in a cosmid pHU1 as defined in Claim 1 or a cosmid pHU2 as defined in Claim 2 into free-living cells of the strain so that they are converted to Hup$^+$ phenotype; and inoculating such plants of the particular Leguminosae species with such cells having Hup$^+$ phenotype.

13. The process of Claim 12, wherein: the *Rhizobium* species is *Rhizobium japonicum*; and the Leguminosae species is *Glycine max*.

14. The process of Claim 12 comprising inserting a pHU1 cosmid as defined in Claim 1, or a pHU2 cosmid as defined in Claim 2 into the free-living cells.

15. A recombinant DNA molecule comprising a first segment containing HU DNA as defined in Claim 1 present in cosmid pHU1 as defined in Claim 1 or cosmid pHU2 as defined in Claim 2 flanked by second and third DNA segments that are not the DNA that flanks the naturally occurring HU DNA.

**Patentansprüche**

1. Cosmid pHU1, welches ein Cosmid ist, das durch rekombinante DNA-Techniken hergestellt ist und HU-DNA enthält, die *hup*-Gene oder Teile von *hup*-Genen enthält, wobei das Cosmid pHU1 in der Kultur von *Escherichia coli* HB101 mit einer Hinterlegungszugriffsnummer ATCC 39195 vorhanden ist.

2. Cosmid pHU2, welches ein Cosmid ist, das durch rekombinante DNA-Techniken hergestellt ist und HU-DNA enthält, wie in Anspruch 1 definiert, wobei das Cosmid pHU2 in der Kultur von *Escherichia coli* HB101 mit der Hinterlegungszugriffsnummer ATCC 39196 vorhanden ist.

3. Replikon, das in einer *Rhizobium*-Spezies gehalten werden kann, die für die Stickstoff-Fixierung in Leguminosen nützlich ist, wobei das Replikon ein Klonierungsvektor ist, der mit HU-DNA, wie in Anspruch 1 definiert, verbunden ist, die für Wasserstoffaufnahmeaktivität notwendig ist, wobei die HU-DNA diejenige HU-DNA ist, die in Cosmid pHU1, wie in Anspruch 1 definiert, oder in Cosmid pHU2, wie in Anspruch 2 definiert, vorhanden ist.

4. *Escherichia coli* HB101, welches das Cosmid pHU1, wie in Anspruch 1 definiert, enthält, mit der Hinterlegungszugriffsnummer ATCC 39195.

5. Biologisch reine Kultur des *Escherichia coli* HB101 gemäß Anspruch 4.

6. *Escherichia coli* HB101, welches das Cosmid pHU2, wie in Anspruch 2 definiert, enthält, mit der Hinterlegungszugriffsnummer ATCC 39196.

7. Biologisch reine Kultur des *Escherichia coli* HB101 gemäß Anspruch 6.

8. Verfahren zum Herstellen eines Replikons, das Gene oder Teile von Genen enthält, die für Wasserstoffgasaufnahmevermögen (Hup) kodieren, und das zur Einführung in eine stickstoff-fixierende *Rhizobium*-Spezies geeignet ist, wobei das Verfahren folgendes umfaßt: Verbinden von DNA-Fragmenten, die *hup*-Gene oder Teile von *hup*-Genen enthalten, von der Art, wie sie in einem Cosmid pHU1, wie in Anspruch 1 definiert, oder in einem Cosmid pHU2, wie in Anspruch 2 definiert, vorhanden sind, mit Replikons, die in einem Hup$^-$-Stamm einer *Rhizobium*-Spezies existieren können; Einführen der Replikons in Zellen des Hup$^-$-Stammes; Identifizieren, welche Proben des Hup$^-$-Stamms in Hup$^+$ überführt worden sind; und Isolieren der Replikons.

9. Verfahren zum Bereitstellen eines aktiven wasserstoffoxidierenden Systems in freilebenden Zellen eines Stamms einer *Rhizobium*-Spezies, der ein solches System fehlt und die für die Stickstoff-Fixierung in einer Leguminose nützlich ist, welches das Einführen eines pHU1-Cosmids, wie in Anspruch 1 definiert, oder eines pHU2-Cosmids, wie in Anspruch 2 definiert, in die Zellen umfaßt.

10. Verfahren nach Anspruch 9, wobei das Verfahren weiterhin den Schritt des Identifizierens einer Kolonie von *Rhizobium japonicum* mit Wasserstoffaufnahmeaktivität aus einer Gruppe von Kolonien umfaßt, von denen einige keine Wasserstoffaufnahmeaktivität aufweisen, wobei dieser Schritt folgendes umfaßt: Replizieren von Kolonien der Gruppe auf eine Oberfläche eines porösen, sterilen Trägermaterials; Inkubieren von Zellen auf der Oberfläche unter Bedingungen, die ihre Hydrogenase dereprimieren; Behandeln des Trägermaterials mit einer Methylenblau-Indikatorlösung, die Inhibitoren der endogenen Atmung enthält; Halten des Trägermaterials mit anhängenden Kolonien in einer $H_2$-Atmosphäre,

woraufhin Kolonien mit Wasserstoffaufnahmeaktivität das Methylenblau zu seiner Leuko-Form reduzieren; Extrahieren und/oder Klonieren von Hu-DNA, wie in Anspruch 1 definiert, aus Kolonien mit Wasserstoffaufnahmeaktivität und Herstellen eines Cosmids pHU1, wie in Anspruch 1 definiert, oder eines Cosmids pHU2, wie in Anspruch 2 definiert, vor dem Einführen des pHU1- oder pHU2-Cosmids in die Zellen.

11. Verfahren nach Anspruch 10, wobei: das Trägermaterial Filterpapier umfaßt; während des Inkubierens das Filterpapier mit der Kolonieseite nach oben auf Platten von Repaske-Medium gelegt werden; des Inkubieren in einem geschlossenen Behälter abläuft, wobei die Atmosphäre anfänglich etwa 5% $H_2$, 5% $CO_2$, 0,7% $O_2$ und der Rest $N_2$ ist, wobei man zuläßt, daß der $O_2$-Gehalt während des Inkubierens bis unter 0,1% absinkt; der $O_2$-Gehalt danach durch Zugaben von $O_2$ bei etwa 0,2% gehalten wird; die Methylenblau-Indikatorlösung 200 mM Jodessigsäure, 200 mM Malonsäure, 10 mM Methylenblau, 50 mM $KH_2PO_4$, 2,5 mM $MgCl_2$ und eine Menge an KOH enthält, die ausreicht, um den pH auf 5,6 einzustellen.

12. Verfahren zum Steigern der Stickstoff-Fixierung in Leguminosen, die stickstoff-fixierende Baktieren beherbergen, welches normalerweise ein aktives wasserstoffoxidierendes System fehlt, wobei das Verfahren folgendes umfaßt: Identifizieren eines Stamms einer *Rhizobium*-Spezies, welches die gewünschte stickstoff-fixierende Aktivtät aufweist, wenn sie in den Wurzeln von Pflanzen einer bestimmten Leguminosen-Spezies vorhanden ist; Einführen von DNA, die wasserstoffaufnehmendes Genmaterial der Art enthält, die in einem Cosmid pHU1, wie in Anspruch 1 definiert, oder einem Cosmid pHU2, wie in Anspruch 2 definiert, vorhanden ist, in freilebenden Zellen des Stamms, so daß sie in den Hup$^+$-Phänotyp überführt werden; und Beimpfen solcher Pflanzen der besonderen Leguminosen-Spezies mit solchen Zellen mit Hup$^+$-Phänotyp.

13. Verfahren nach Anspruch 12, wobei: die *Rhizobium*-Spezies *Rhizobium japonicum* ist; und die Leguminosen Spezies *Glycine max* ist.

14. Verfahren nach Anspruch 12, welches das Einführen eines pHU1-Cosmids, wie in Anspruch 1 definiert, oder eines pHU2-Cosmids, wie in Anspruch 2 definiert, in die freilebenden Zellen umfaßt.

15. Rekombinantes DNA-Molekül, welches ein erstes Segment umfaßt, das HU-DNA, wie in Anspruch 1 definiert, enthält, das in Cosmid pHU1, wie in Anspruch 1 definiert, oder Cosmid pHU2, wie in Anspruch 2 definiert, vorhanden ist, flankiert von zweiten und dritten DNA-Segmenten, die nicht die DNA sind, welche die natürlich vorkommende HU-DNA flankieren.

**Revendications**

1. Cosmid pHU1, qui est un cosmide préparé par des techniques d'ADN recombinant et contentant de l'ADN HU, lequel ADN contient des gènes *hup* ou des portions de gènes *hup*, ce cosmide pHU1 étant présent dans la culture d'*Escherichia coli* HB101 dont le numéro d'accès au dépôt est ATCC 39195.

2. Cosmide pHU2, qui est un cosmide préparé par des techniques d'ADN recombinant et contenant de l'ADN HU tel que défini dans la revendication 1, ce cosmide pHU2 étant présent dans la culture d'*Escherichia coli* HB101 dont le numéro d'accès au dépôt est ATCC 39196.

3. Replicon susceptible d'être maintenu dans une espèce de Rhizobium qui est utile pour la fixation d'azote dans les plantes légumineuses, dans lequel le replicon est un vecteur de clonage raccordé à de l'ADN HU tel que défini dans la revendication 1 nécessaire pour l'activité de fixation d'hydrogène, l'ADN HU étant l'ADN HU présent dans le cosmide pHU1 conforme à la revendication 1 ou dans le cosmid pHU2 conforme à la revendication 2.

4. *Escherichia coli* HB101 contenant le cosmide pHU1 conforme à la revendication 1, dont le numéro d'accès au dépôt est ATCC 39195.

5. Culture biologiquement pure de l'*Escherichia coli* HB101 selon la revendication 4.

6. *Escherichia coli* HB101 contenant le cosmide pHU2 conforme à la revendication 2, dont le numéro d'accès au dépôt est ATCC 39196.

7. Culture biologiquement pure de l'*Escherichia coli* HB101 selon la revendication 6.

8. Procédé de préparation d'un replicon qui contient des gènes ou portions de gènes codant pour la capacité de fixation d'hydrogène gazeux (Hup) et qui est adapté à être introduit dans une espèce de Rhizobium fixant l'azote, le procédé comprenant: la jonction de fragments d'ADN qui contiennent des gènes *hup* ou des portions de gènes *hup* du type présent dans un cosmide pHU1 conforme à la revendication 1 ou dans un cosmide pHU2 conforme à la revendication 2, à des replicons qui peuvent exister dans une souche Hup$^-$ d'une espèce de Rhizobium; l'introduction des replicons dans des cellules de la souche Hup$^-$; l'identification des échantillons de souche Hup$^-$ qui ont été transformés en Hup$^+$; et l'isolement du replicon.

9. Procédé de réalisation d'un système oxydant l'hydrogène actif dans des cellules vivant à l'état libre d'une souche d'une espèce de Rhizobium qui est dépourvue d'un tel système et qui est utile pour la fixation d'azote dans une plante légumineuse, comprenant l'insertion d'un cosmide pHU1 conforme à la revendication 1 ou d'un cosmide pHU2 conforme à la revendication 2 dans les cellules.

10. Procédé selon la revendication 9, qui comprend en outre le stade d'identification d'une colonie de *Rhizobium japonicum* ayant une activité de fixation de l'hydrogène dans un groupe de colonies dont certaines n'ont pas d'activité de fixation de l'hydrogène, lequel stade comprend: la réplication de colonies du groupe sur la surface d'un matériau de support poreux, stérile; l'incubation des cellules sur la surface dans des conditions qui libèrent leur hydrogénase; le traitement du matériau de support par une solution d'indicateur au bleu de méthylène contenant des inhibiteurs de la respiration endogène; le maintien du matériau de support avec les colonies fixées dans une atmosphère de $H_2$, les colonies ayant une activité de fixation de l'hydrogène réduisant ainsi le bleu de méthylène en sa forme leuco; l'extraction et/ou le clonage de l'ADN HU tel que défini dans la revendication 1, des colonies ayant une activité de fixation d'hydrogène, et la préparation d'un cosmide pHU1 conforme à la revendication 1 ou d'un cosmide pHU2 conforme à la revendication 2, avant l'insertion du cosmide pHU1 ou pHU2 dans les cellules.

11. Procédé selon la revendication 10, dans lequel le matériau de support comprend du papier filtre; au cours de l'incubation, le papier filtre est placé le côté colonie au-dessus sur des plaques de milieu de Repaske; l'incubation se produit dans un récipient fermé dans lequel l'atmosphère est initialement constituée d'environ 5% de $H_2$, 5% de $CO_2$, 0,7% de $O_2$, et le reste de $N_2$, en laissant le taux d'$O_2$ descendre au-dessous de 0,1% au cours de l'incubation; le taux de $O_2$ est ensuite maintenu aux environs de 0,2% par des additions d'$O_2$; la solution d'indicateur au bleu de méthylène contient 200 mM d'acide iodoacétique, 200 mM d'acide malonique, 10 mM de bleu de méthylène, 50 mM de $KH_2PO_4$, 2,5 mM de $MgCl_2$, et une quantité de KOH suffisante pour régler le pH à 5,6.

12. Procédé pour augmenter la fixation d'azote dans des plantes légumineuses qui abritent des bactéries fixant l'azote normalement dépourvues de système d'oxydation de l'hydrogène, le procédé comprenant: l'identification d'une souche d'une espèce de Rhizobium qui a l'activité de fixation d'azote désirée lorsqu'elle est présente dans les racines de plantes d'une espèce de Leguminosae particulière; l'introduction d'ADN contenant du matériau de gènes de fixation d'hydrogène du type présent dans un cosmide pHU1 conforme à la revendication 1 ou dans un cosmide pHU2 conforme à la revendication 2, dans des cellules vivant à l'état libre de la souche, de telle sorte qu'elles sont transformées en le phénotype Hup$^+$; et l'inoculation de ces plantes de l'espèce de Leguminosae particulière avec ces cellules ayant un phénotyp Hup$^+$.

13. Procédé selon la revendication 12, dans lequel l'espèce de *Rhizobium* est le *Rhizobium japonicum*; et l'espèce de Leguminosae est la *Glycine max*.

14. Procédé selon la revendication 12, comprenant l'insertion d'un cosmide pHU1 conforme à la revendication 1 ou d'un cosmide pHU2 conforme à la revendication 2, dans des cellules vivant à l'état libre.

15. Molécule d'ADN recombinant comprenant un premier segment contenant un ADN HU tel que défini dans la revendication 1 présent dans le cosmide pHU1 conforme à la revendication 1 ou dans le cosmide pHU2 conforme à la revendication 2, encadré par un second et un troisième segments d'ADN qui ne sont pas l'ADN qui encadre l'ADN HU naturel.